# EUROPEAN PATENT APPLICATION

(11) **EP 1 426 359 A1**
(43) Date of publication of application: **09.06.2004**
(21) Application number: 02760683.9
(22) Date of filing: 21.08.2002
(51) Int. Cl.: C07D 211/34

(54) **PROCESS FOR PREPARATION OF PIPERIDIN-2-YLACETIC ACID**

(30) Priority: 21.08.2001 JP 2001250533
(71) Applicant: FUJISAWA PHARMACEUTICAL CO., LTD., Osaka-shi Osaka 541-8514 (JP)
(72) Inventor: ZANKA, A., c/o FUJISAWA PHARMACEUTICAL CO., LTD., Osaka-shi, Osaka 541-8514 (JP); OKAMOTO, T., c/o FUJISAWA PHARMACEUTICAL CO., LTD., Osaka-shi, Osaka 541-8514 (JP); HASHIMOTO, N., c/o FUJISAWA PHARMACEUTICAL CO.,LTD, Osaka-shi, Osaka 541-8514 (JP); GOTO, S., c/o FUJISAWA PHARMACEUTICAL CO., LTD., Osaka-shi, Osaka 541-8514 (JP)
(74) Representative: Gille Hrabal Struck Neidlein Prop Roos
(86) International application number: PCT/JP2002/008422
(87) International publication number: WO 2003/016278

(57) **Abstract**

Piperidin-2-ylacetic acid (II) is efficiently and safely produced in industrial scale by treating piperidin-2-ylethanol (I) with 2,2,6,6-tetramethylpiperidin-1-oxyl, sodium hypochlorite and sodium chlorite in a two phase solvent comprising an organic solvent and water without using strongly toxic chromium trioxide.

## Description

### [Technical field]

The present invention relates to a new process for preparing piperidin-2-ylacetic acid (II) having the formula (II). 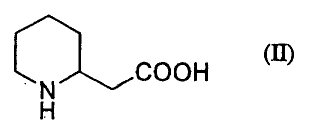 Piperidin-2-ylacetic acid (II) is useful, for example, as an intermediate for manufacturing a pyrazolopyridine compound (III) having the formula (III): 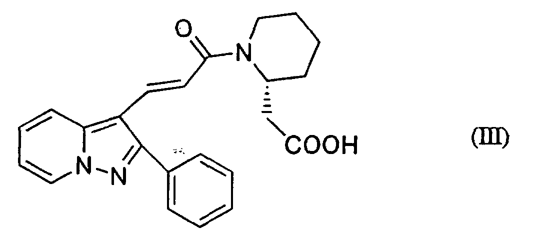 which possesses an excellent adenosine antagonism activity [Japanese Patent Kokai No. Hei 05-112566].

### [Background art]

A method for preparing piperidin-2-ylacetic acid (II) by subjecting piperidin-2-ylethanol (I) having the formula (I): 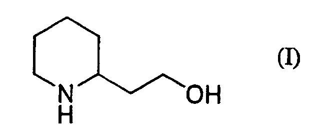 to Jones oxidation is known [Knight D.W, et al., J. Chem. Soc. Perkin Trans. I, 1994, 2903].

According to the above oxidation, there gives an advantage that ethyl alcohol moiety at the side chain is selectively oxidized to acetic acid without oxidation of the nitrogen atom in the piperidine ring. To the contrary, it is unsuitable for industrial-scale production, because the oxidation reaction requires to use strongly toxic chromium trioxide as an oxidizing agent, and further gives a waste liquid containing chromium after the reaction.

Meanwhile, a method is known, in which a primary alcohol is oxidized to a carboxylic acid by treating the alcohol in acetonitrile with sodium hypochlorite (NaOCl) in the presence of 2,2,6,6-tetramethylpiperidin-1-oxyl (TEMPO) [Nooy, A.E., et al., Synthesis, 1996, 1153]. However, this method is unsatisfactory for reaction efficacy, because it requires a largely excessive amount of sodium hypochlorite.

In addition, a two-step reaction is known, in which a primary alcohol is oxidized by the Swem oxidation using dimethyl sulfoxide and oxalyl chloride to yield an aldehyde, followed by oxidation of the resulting aldehyde to the carboxylic acid by using sodium chlorite (NaClO₂) [Lindgren, B.O., et al., Acta Chem. Scand., 1973, 2, 888]. However, this method is complicate in post-processing, and is difficult to apply it to large-scale synthesis.

Furthermore, a method is also known, in which a primary alcohol substituted with an aromatic hydrocarbon ring, a hetero ring or an alkynyl group is oxidized to the corresponding carboxylic acid by treating the alcohol with sodium hypochlorite and sodium chlorite in the presence of TEMPO in a two phase solvent comprising acetonitrile and a low pH range buffer solution [Mangzhu Zhao, et al., J. Org. Chem., 1999, 64, 2564]. According to this method, the alcohol is efficiently oxidized to the carboxylic acid, but careful attention must be paid during operation for adding sodium hypochlorite and sodium chlorite, because they must be added simultaneously notwithstanding the danger of furious reaction of sodium hypochlorite with sodium chlorite. Moreover, keeping the reaction liquid under an acidic condition is required to proceed the reaction, and accordingly a large amount of an acidic buffer solution is necessary. Therefore, it is difficult to apply this method to large-scale synthesis.

### [Disclosure of invention]

The inventors of the present invention have concentrated their study to develop a method which is suitable for producing piperidin-2-ylacetic acid (I) on industrial scale by the oxidation of piperidin-2-ylethanol (I) or its salt, and have found that the objective piperidin-2-ylacetic acid (II) can be produced efficiently, safely and industrially without protecting NH of the piperidine ring by treating piperidin-2-ylethanol (I) or its salt with sodium hypochlorite (NaOCl) and sodium chlorite (NaClO₂) in the presence of 2,2,6,6-tetramethylpiperidin-1-oxyl (TEMPO) in a two phase solvent comprising an organic solvent and water, and then have completed the present invention.

### [Best mode for carrying out invention]

The present invention is carried out by treating piperidin-2-ylethanol (I) of the formula (I): 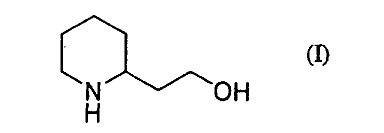 or its salt with sodium hypochlorite and sodium chlorite in the presence of 2,2,6,6-tetramethylpiperidin-1-oxyl (TEMPO) in a two phase solvent comprising an organic solvent and water to obtain piperidin-2-ylacetic acid (II) of the formula (II).

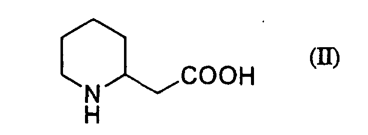

Piperidin-2-ylethanol (I) of the formula (I) and piperidin-2-ylacetic acid (II) of the formula (II) have an asymmetric carbon atom at 2-position of the piperidine ring, and therefore piperidin-2-ylethanol (I) and piperidin-2-ylacetic acid (II) include (2S) form, (2R) form and a mixture thereof, respectively.

Piperidin-2-ylethanol (I) can be prepared according to a method described in prior arts [for example, Toy M. S. et al., J. Am. Chem. Soc., 1960, 82, 2613].

As a salt of piperidin-2-ylethanol (I), a salt with an inorganic base, for example, with an alkali metal (sodium, potassium or the like) or alkaline earth metal (calcium, magnesium or the like); or an inorganic acid addition salt, for example, hydrochloride, hydrobromide, hydroiodide, sulfate, phosphate or the like; can be exemplified. The salt with an inorganic base is preferable. These salts of piperidin-2-ylethanol (I) can be prepared by treating piperidin-2-ylethanol (I) with an appropriate base or an acid in accordance with the conventional method.

The amount of each reagents to be used in the reaction is not limited. However, TEMPO to be used is about 0.001 - 0.1 mol, preferably about 0.01 - 0.02 mol of TEMPO with respect to 1 mol of piperidin-2-ylethanol (I). Sodium hypochlorite is considered to be required to use approximate equimolar amount, because sodium hypochlorite is used to oxidize the piperidn-2-ylethanol (I) to the aldehyde. In the present invention, however, equimolar or less amount of sodium hypochlorite may be enough, because sodium chlorite is consumed as the reaction proceeds but sodium hypochlorite is produced as a by-product in the reaction system. Accordingly, sodium hypochlorite may use about 0.1 - 5.0 mol, preferably, about 0.5 - 2.0 mol.

Sodium chlorite is considered to be required to use approximate equimolar amount, because it oxidizes the aldehyde to piperidin-2-ylacetic acid (II). However, since a part of the aldehyde is oxidized to piperidin-2-ylacetic acid (II) also by sodium hypochlorite, the amount of sodium chlorite may be equimolar or less. Accordingly, sodium chlorite may use about 0.5 - 5.0 mol, but preferably, about 1.0 - 2.0 mol.

The organic solvent used in the present method forms two-phase system with water, and accordingly, the solvent is not substantially miscible with water. From the industrial view point, for example, ethyl acetate, methylene chloride or the like is preferable, and toluene, acetonitrile or the like can also be used. These organic solvents are used alone or in combination with two or more of such solvents. Although the ratio of the organic solvents and water is not specifically restricted, about 0.1 - 10 volumes of water, preferably about 0.1 - 3 volumes of water is usually used with respect to one volume of the organic solvent.

According to the method of the present invention, it is preferable that piperidin-2-ylethanol (I) or its salt is treated with sodium hypochlorite in the presence of TEMPO in a two phase solvent comprising an organic solvent and water under neutral or basic conditions and then treated with sodium chlorite under acidic conditions to obtain piperidin-2-ylacetic acid (II).

In the above method, the treatment of piperidin-2-ylethanol (I) or its salt with sodium hypochlorite in the presence of TEMPO is carried out by mixing piperidin-2-ylethanol (I) or its salt, TEMPO (catalytic amount) and sodium hypochlorite in a two phase solvent comprising an organic solvent and water under the condition that the aqueous phase is basic, preferably about pH 7 - 10, more preferably about pH 8 - 9. Preferably, sodium hypochlorite is added dropwise to the mixture of piperidin-2-ylethanol (I) or its salt and TEMPO (catalytic amount) in a two phase solvent comprising an organic solvent and water in about 5 minutes - 1 hour, and then the reaction mixture is stirred at a temperature under cooling to ambient temperature, preferably at about 1 - 10 °C, for about ten minutes - a few hours.

As a base to be used for adjusting the pH value in the above treatment, an inorganic base or organic base such as an alkali metal carbonate, alkali metal hydrogencarbonate, tri(lower)alkylamine, pyridine, piperidine, N-(lower)alkylmorpholine, N,N-di(lower)alkylbenzylamine or the like can be exemplified. Since piperidin-2-ylethanol (I) is a basic substance, the above-mentioned base or an acid can be used in an amount to give a desired pH value. As an acid to be used for adjusting the pH value, an inorganic acid, for example, hydrochloric acid, hydrobromic acid, sulfuric acid, hydrogen chloride or hydrogen bromide, etc. and an organic acid, for example, formic acid, acetic acid, propionic acid, trichloroacetic acid or trifluoroacetic acid etc. can be mentioned.

The next treatment with sodium chlorite is carried out, after adjusting the aqueous phase of the two phase solvent to be acidic, preferably to about pH 3 - 6, more preferably to about pH 5, by adding dropwise sodium chlorite to the resultant mixture at about 20 - 50 °C, preferably at about 25 - 35 °C, preferably in about 5 minutes - 1 hour, and then by stirring the resultant reaction mixture at about 15 - 50 °C, preferably at about 15 - 25 °C, for about 10 minutes - 50 hours, preferably for about 3 - 24 hours.

As an acid to be used for adjusting the pH value in the above treatment, the above mentioned inorganic acids, for example, hydrochloric acid, hydrobromic acid, sulfuric acid, hydrogen chloride or hydrogen bromide, etc., and organic acids, for example, formic acid, acetic acid, propionic acid, trichloroacetic acid or trifluoroacetic acid, etc. can be mentioned. The acid is used in a necessary amount to obtain a desired pH value.

The piperidin-2-ylacetic acid (II) thus obtained can be isolated and purified from the reaction mixture by the conventional manners, for example, extraction, precipitation, removal of solvent by distillation, fractional crystallization, recrystallization, chromatography and the like.

It is believed that the reaction according to the method of the present invention proceeds as follows.

At first, a part of piperidin-2-ylethanol (I) is oxidized to the corresponding aldehyde by oxidized-form TEMPO, which is produced from TEMPO and sodium hypochlorite, in an organic solvent phase under a neutral to basic condition. Then, the aldehyde is transferred to the aqueous phase under acidic condition and oxidized by sodium chlorite to give piperidin-2-ylacetic acid (II) while sodium hypochlorite is produced as a by-product. Sodium hypochlorite thus produced as a by-product reacts with TEMPO to give the oxidized-form TEMPO and oxidizes the pipderidin-2-ylethanol (I) remaining in the organic phase to give the aldehyde. The aldehyde is oxidized to piperidin-2-ylacetic acid (II) by sodium chlorite in the aqueous phase under an acidic condition.

According to the method of the present invention, since the reaction is carried out in the two phase solvent comprising organic solvent and water, the above two-step oxidation reaction continuously proceeds in the same reactor without vigorous reaction between sodium hypochlorite and sodium chlorite. Accordingly, the objective piperidin-2-ylacetic acid (II) can be produced safely and efficiently. This method is suitable for industrial-scale synthesis, because the reaction can be carried out by using the raw material and the reagents in high concentration.

The piperidin-2-ylacetic acid (II) thus obtained can be converted to a pyrazolopyridine compound (III) in accordance with a method described in, for example, Japanese Patent Kokai No. Hei 05-112566.

The method of the present invention is explained by the following Examples, but the scope of the invention is not limited to these Examples.

### Example 1

Ethyl acetate (150 ml) was added to a mixture of piperidin-2-ylethanol (12.92 g, 100 mmol), potassium bromide (1.19 g) and water (20 ml). The mixture was adjusted to pH 8.6 with 35% hydrochloric acid at 5 °C, and then 2,2,6,6-tetramethylpiperidin-1-oxyl (TEMPO 0.31 g, 2 mmol) was added thereto. Then, 12% sodium hypochlorite aqueous solution (125 mmol) was added dropwise to the mixture at 5 °C in 20 minutes while adjusting the pH of the mixture to 8.0 - 9.0 with 35% hydrochloric acid. After the mixture was stirred at room temperature for 30 minutes, the pH of the mixture was adjusted to 5.0 with 35% hydrochloric acid. Then 25% sodium chlorite aqueous solution (125 mmol) was added dropwise to the mixture in 30 minutes while keeping the reaction mixture at 27 - 33 °C. The reaction mixture was stirred at room temperature for 16 hours. After the reaction was completed, the reaction mixture was distilled under a reduced pressure to give an oily product containing piperidin-2-ylacetic acid (11.2 g, yield: 78%).

Piperidin-2-ylacetic acid was identified as the methyl ester hydrochloride.
¹H NMR (200 MHz, DMSO-d6) δ (ppm): 1. 50-1.56 (m, 2H), 1.66-1.81 (m, 4H), 2.68 (dd, 1H, J=8.1, 4.1 Hz), 2.75-3.00 (m, 1H), 2.92 (dd, 1H, J=8.1, 2.6 Hz, 3.19-3.22 (m, 1H), 3.28-3.47 (m, 1H), 3.65 (s, 3H), 9.35 (brs, 2H).

### Example 2

Ethyl acetate (150 ml) was added to a mixture of R-(-)-piperidin-2-ylethanol (12.92 g, 100 mmol), potassium bromide (1.19 g) and water (0.5 M, 20 ml). The mixture was adjusted to pH 8.6 at 5 °C with 35% hydrochloric acid, and then 2,2,6,6-tetramethylpiperidin-1-oxyl (0.31 g, 2 mmol) was added thereto. Then, 12% sodium hypochlorite aqueous solution (125 mmol) was added dropwise at 5 °C in 20 minutes while adjusting the pH value of the mixture to 8.0 - 9.0 with 35% hydrochloric acid. The mixture was stirred at room temperature for 30 minutes and then adjusted to pH 5.0 with 35% hydrochloric acid. Then, 25% sodium chlorite aqueous solution (125 mmol) was added dropwise in 30 minutes while keeping the reaction mixture at 27 - 33 °C, and the resultant mixture was stirred at room temperature for 16 hours. After the reaction was completed, the reaction mixture was distilled under a reduced pressure to give an oily product containing R-(-)-piperidin-2-ylacetic acid (10.9 g, yield: 76%).

### [Possibility of industrial use]

According to the present invention, piperidin-2-ylacetic acid (II) can efficiently be produced from piperidin-2-ylethanol (I) without using strongly toxic chromium trioxide and also without protecting NH of the piperidine ring.

In addition, according to the present invention, the objective piperidin-2-ylacetic acid (II) can be efficiently produced, because the reaction is carried out in a two phase solvent comprising an organic solvent and water, and thereby the oxidation reaction from the alcohol to the aldehyde and the oxidation reaction from the aldehyde to the carboxylic acid continuously proceed in the same reactor.

Further, according to the present invention, piperidin-2-ylethanol (I) used as the starting material can be efficiently converted to piperidin-2-ylacetic acid (II), because sodium hypochlorite is produced as a by-product from sodium chlorite, which serves as an oxidizing agent in the oxidation reaction from an aldehyde to a carboxylic acid, and then the unreacted piperidin-2-ylethanol (I) remaining in the reaction mixture is oxidized to the aldehyde by sodium hypochlorite thus produced.

Furthermore, according to the present invention, piperidin-2-ylacetic acid (II) can be safely produced, because the furious reaction of sodium hypochlorite and sodium chlorite can be avoided by using them in a two phase solvent comprising an organic solvent and water, though these sodium hypochlorite and sodium chlorite furiously react with each other.

## Claims

1. A process for preparing piperidin-2-ylacetic acid of the formula (II): 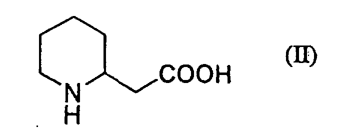 which is **characterized in that** piperidin-2-ylethanol of the formula (I): 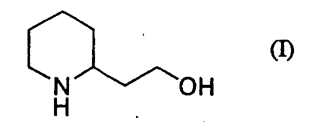 or its salt is treated with 2,2,6,6-tetramethylpiperidin-1-oxyl, sodium hypochlorite and sodium chlorite in a two phase solvent comprising an organic solvent and water to give piperidin-2-ylacetic acid.

2. The process for preparing piperidin-2-ylacetic acid of the formula (II): 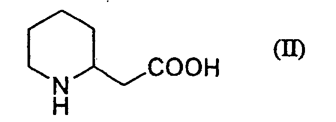 according to claim 1 which is **characterized in that** piperidin-2-ylethanol of the formula (I): 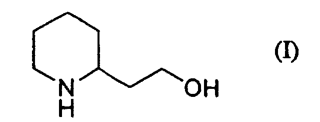 or its salt is treated with sodium hypochlorite in the presence of 2,2,6,6-tetramethylpiperidin-1-oxyl under a neutral to basic condition and then treated with sodium chlorite under an acidic condition to give the piperidin-2-ylacetic acid.

3. The process according to claims 1 or 2 wherein the organic solvent is ethyl acetate, toluene, acetonitrile or methylene chloride.

4. The process according to claims 2 or 3 wherein the treatment with sodium hypochlorite in the presence of 2,2,6,6-tetramethylpiperadin-1-oxyl is carried out at about pH 7 - 10.

5. The process according to any one of claims 2 to 4 wherein the treatment with sodium chlorite is carried out at about pH 3 - 6.
